# EUROPEAN PATENT SPECIFICATION

(11) **EP 4 514 453 B1**
(45) Date of publication and mention of the grant of the patent: **14.10.2026**
(21) Application number: 23797503.2
(22) Date of filing: 25.04.2023
(51) Int. Cl.: A61N 1/36, A61N 1/05

(54) **SYSTEMS FOR CONCURRENTLY DELIVERING HIGH FREQUENCY AND LONG PULSE WIDTH ELECTRICAL STIMULATION**
SYSTEME ZUR GLEICHZEITIGEN ABGABE VON ELEKTRISCHER HOCHFREQUENZ- UND LANGER IMPULSBREITE-STIMULATION
SYSTÈMES POUR DÉLIVRER SIMULTANÉMENT UNE STIMULATION ÉLECTRIQUE À HAUTE FRÉQUENCE ET À GRANDE LARGEUR D'IMPULSION

(30) Priority: 25.04.2022 US 202263334270 P
(43) Date of publication of application: 05.03.2025
(73) Proprietor: Nevro Corporation, Redwood City, CA 94065 (US)
(72) Inventor: LEE, Dongchul, Agua Dulce, CA 91390 (US); BRADLEY, Kerry, Glendale, CA 91208 (US)
(74) Representative: Morabito, Sara
(86) International application number: PCT/US2023/066215
(87) International publication number: WO 2023/212581

(56) References cited:
- AU-A1- 2020 220 021
- US-A1- 2016 121 109
- US-A1- 2017 036 029
- US-A1- 2018 056 073
- US-A1- 2020 094 055
- US-A1- 2021 228 881
- US-A1- 2021 379 386
- US-A1- 2022 062 636
- US-B1- 10 682 516

## Description

### CROSS-REFERENCE TO RELATED APPLICATION(S)

The present application claims priority to U.S. Provisional Application No. 63/334,270, filed April 25, 2022.

### TECHNICAL FIELD

The present technology is directed toward electrically modulating nervous tissue to treat a patient condition.

### BACKGROUND

Neurological stimulators have been developed to treat pain, movement disorders, functional disorders, spasticity, cancer, cardiac disorders, and various other medical conditions. Implantable neurological stimulation systems generally have an implantable signal generator and one or more leads that deliver electrical pulses to neurological tissue or muscle tissue. For example, several neurological stimulation systems for spinal cord stimulation (SCS) have cylindrical leads that include a lead body with a circular cross-sectional shape and one or more conductive rings (e.g., contacts) spaced apart from each other at the distal end of the lead body. The conductive rings operate as individual electrodes and, in many cases, the SCS leads are implanted percutaneously through a needle inserted into the epidural space, with or without the assistance of a stylet. In other systems, the electrodes are carried by a paddle that is implanted via a laminotomy.

Once implanted, the signal generator applies electrical pulses to the electrodes, which in turn modify the function of the patient's nervous system, such as by altering the patient's responsiveness to sensory stimuli and/or altering the patient's motor-circuit output. In SCS therapy for the treatment of pain, for example, the signal generator applies electrical pulses to the spinal cord via the electrodes.

US 2021/228881 and US 2017/036029 discloses patient treatment system known in the art.

### BRIEF DESCRIPTION OF THE DRAWINGS

Figure 1 is a partially schematic illustration of an implantable spinal cord modulation system positioned at a patient's spine to deliver therapeutic signals in accordance with some embodiments of the present technology.
Figure 2 is a partially schematic, cross-sectional illustration of a patient's spine, illustrating representative locations for implanted lead bodies in accordance with some embodiments of the present technology.
Figure 3 is a schematic illustration of a representative lead body suitable for providing modulation to a patient in accordance with some embodiments of the present technology.
Figures 4A-4C are schematic illustrations of representative patterns for administering a first electrical signal and a second electrical signal using the lead body shown in Figure 3 and in accordance with some embodiments of the present technology.
Figure 5 is a line graph comparing neural activity of certain neurons during various stimuli in accordance with some embodiments of the present technology.
Figure 6 is a line graph comparing neural activity of the neurons measured in Figure 5 during various additional stimuli in accordance with some embodiments of the present technology.
Figures 7A and 7B are schematic illustrations of select patterns for administering certain stimuli that were used during the experimental procedures that obtained the data shown in Figure 6.
Figure 8 is a flowchart of a method of treating a patient in accordance with some embodiments of the present technology.

### DETAILED DESCRIPTION

The present invention is defined by the appended claims. Further aspects, embodiments and examples described hereinafter are only of exemplary nature and are presented for better understanding the present invention.

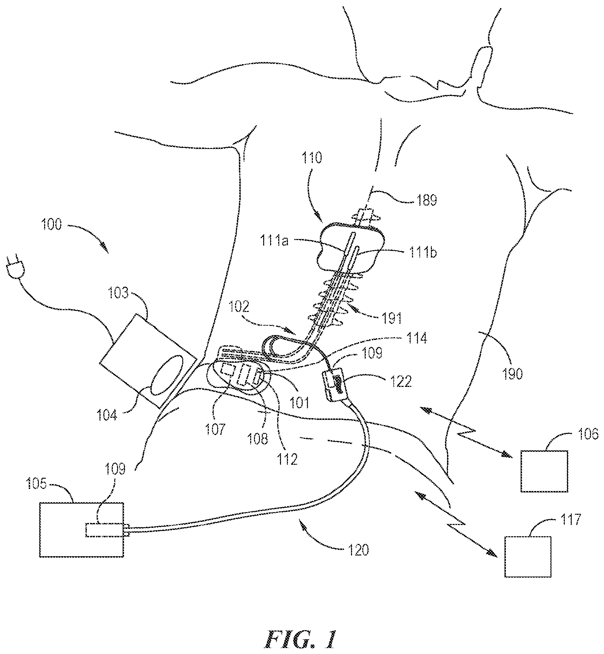

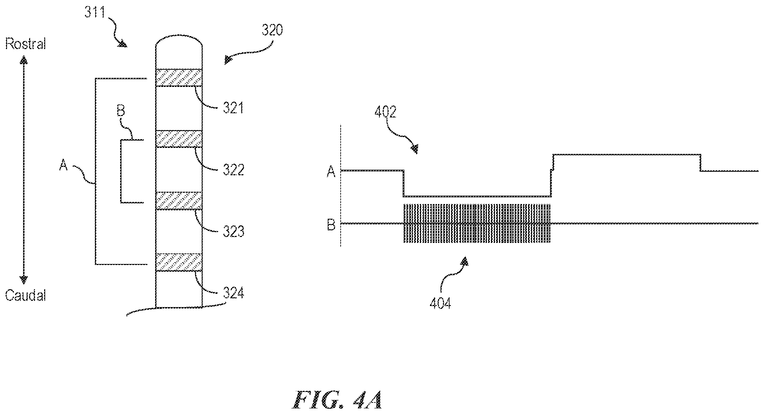

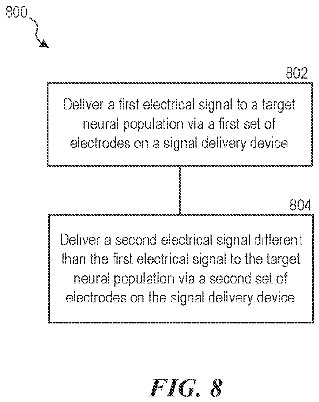

This Detailed Description includes the following headers and sections. which are provided for convenience only and do not affect the scope or meaning of the claimed present technology:
- Definitions of selected terms are provided under Heading 1.0 ("Definitions");
- General aspects of the present technology are described below under Heading 2.0 ("Overview of Present Technology");
- Representative treatment systems and their characteristics are described under Heading 3.0 ("System Characteristics") with reference to Figures 1-3;
- Various patterns of administering electrical stimulation in accordance with the present technology are described under Heading 4.0 ("Representative Embodiments of the Present Technology") with reference to Figures 4A-7B;
- Representative methods of treating patients by suppressing or enhancing neural activity are described under Heading 5.0 ("Methods for Suppressing or Enhancing Neural Activity") with reference to Figure 8;
- Representative clinical applications of the present technology are described under Heading 6.0 ("Representative Clinical Applications"); and
- Representative examples are described under Heading 7.0 ("Representative Examples").

### 1.0 Definitions

Unless otherwise stated, the terms "generally," "about," and "approximately" refer to values within 10% of a stated value. For example, the use of the term "about 100" refers to a range of 90 to 110, inclusive. In instances in which relative terminology is used in reference to something that does not include a numerical value, the terms are given their ordinary meaning to one skilled in the art.

As used herein, and unless otherwise noted, the terms "modulate," "modulation," "stimulate," and "stimulation" refer generally to signals that have an inhibitory, excitatory, and/or other effect on a target neural population. Accordingly, a spinal cord "stimulator" can have an inhibitory effect on certain neural populations. Moreover, the use of the terms "suppress" and "inhibit" in relation to a therapy signal's effect on a neuron refers to a reduction in the neuron's firing rate relative to the neuron's baseline firing rate in the absence of the therapy signal, and does not necessarily refer to a complete elimination of action potentials in the neuron. The "baseline" firing rate can refer to the neuron's spontaneous firing rate and/or the firing rate of the neuron in response to an external stimulus other than the therapy signal.

As used herein, the terms "neuromodulation signal", "electrical therapy signal," "electrical signal," "therapy signal," "signal," and other associated terms are used interchangeably and generally refer to an electrical signal that can be characterized by one more parameters, such as frequency, pulse width, and/or amplitude.

As used herein, the term "preferentially" when used in the context of "preferentially" modulating efferent neural activity or afferent neural activity refers to modulating one of efferent neural activity or afferent neural to a greater degree than the other of efferent neural activity or afferent neural activity. As used herein, the term "selectively" when used in the context of "selectively" modulating efferent neural activity or afferent neural activity refers to modulating one of efferent neural activity or afferent neural activity without modulating (e.g., to a patient-detectable degree) the other of efferent neural activity or afferent neural activity.

As used herein, the term "high frequency" when used to describe an electrical signal refers to an electrical signal having a frequency between 1.2 kHz and about 500 kHz, unless specifically stated otherwise. As used herein, the term "low frequency" when used to describe an electrical signal refers to an electrical signal having a frequency of less than 1.2 kHz, unless specifically stated otherwise.

As used herein, the term "pulse width" refers to the width of any phase of a repeating pulse, such as the portion of a pulse at a given polarity, unless explicitly described otherwise. For example, the use of the term pulse width with respect to a signal having bi-phasic pulses can refer to the duration of an anodic pulse phase or a cathodic pulse phase. The use of the term pulse width with respect to a signal having monophasic pulses can refer to the duration of the monophasic pulse phase.

As used herein, the terms "long pulse width signal," "long pulse width electrical signal," and the like refer to electrical signals having individual pulses with a pulse width in a pulse width range of between about 5 milliseconds and about 2 seconds. For example, for biphasic signals, the term "long pulse width signal" refers to a signal having a leading phase of a given polarity with a pulse width between about 5 milliseconds and about 2 seconds. The charge balancing phase following the leading phase does not necessarily have a pulse width between about 5 milliseconds and about 2 seconds. Moreover, while the long pulse width signals generally have a constant or substantially constant amplitude for the duration of a given pulse phase, in some embodiments the amplitude of the individual pulses may vary during a given pulse phase. In some embodiments, the long pulse width signals may emulate a direct current signal by virtue of maintaining a given polarity for a relatively long duration.

As used herein, the use of terms denoting a direction of electrical current flow, such as "first direction," "second direction," "rostral to caudal," and "caudal to rostral," to describe the direction of electrical current flow for biphasic signals refers to the direction of current flow during a leading (e.g., initial) phase of the biphasic electrical signal, and does not necessarily mean that the electrical signal demonstrates the first or second direction of current flow throughout its entire duration. For example, for a biphasic electrical signal having a "first direction" of current flow, the electrical signal has current flow in the first direction during a leading phase of the electrical signal, and may have current flow in the second direction during a charge balancing phase of the electrical signal. Likewise, for a biphasic electrical signal having a "second direction" of current flow, the electrical signal has current flow in the second direction during the leading phase of the electrical signal, and may have current flow in the first direction during the charge balancing phase of the electrical signal.

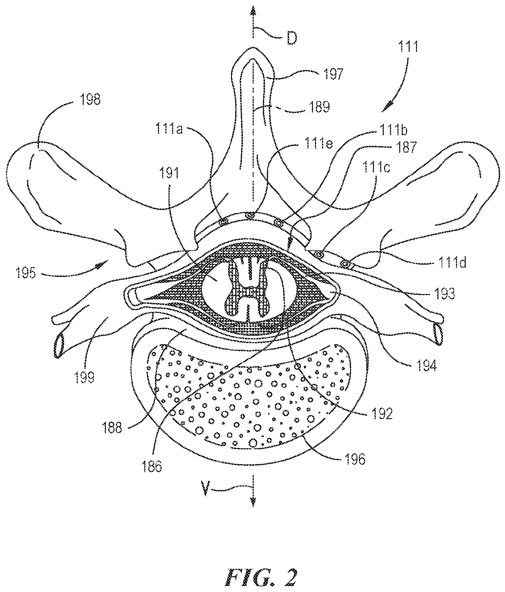

As used herein, "proximate a spinal cord region" refers to the placement of a signal delivery element such that it can deliver electrical stimulation to a neural population located in the spinal cord and/or within the spinal canal. For example, "proximate a spinal cord region" includes, but is not limited to, the relative lead positions described and shown in Figure 2, as well as other positions not expressly described herein.

As used herein, "proximate a target neural population" refers to the placement of a signal delivery element such that it can deliver electrical stimulation to the target neural population. For example, if the target population includes neurons in the spinal cord at a given vertebral level, "proximate the target neural population" includes, but is not limited to, the relative lead positions described and shown in Figure 2 at the given vertebral level, as well as other positions not expressly described herein. As another example, if the target population includes neurons in the patient's cortex (e.g., motor cortex), "proximate the target neural population" includes, but is not limited to, leads positioned in or on the patient's cortex.

### 2.0 Overview of the Present Technology

The present technology is directed generally to electrical stimulation and associated systems and methods. In some embodiments, the present technology includes electrically stimulating a target neural population with multiple electrical signals. For example, a first electrical signal can be applied to the target neural population via a first set of two or more electrodes on a signal delivery element positioned proximate the target neural population, and a second electrical signal can be applied to the target neural population via a second set of two or more electrodes on the same signal delivery element. The first electrical signal and the second electrical signal can have different parameters. For example, the first electrical signal can be a low frequency electrical signal having a frequency under 1.2 kHz, and the second electrical signal can be a high frequency electrical signal having a frequency greater than 1.2 kHz. In some embodiments, the first electrical signal has a relatively long pulse width, such as between about 5 milliseconds and about 2 seconds, such that it approximates a direct current signal.

In some embodiments, electrical stimulation delivered in accordance with the present technology can selectively and/or preferentially modulate neural activity in a direction-specific manner. For example, in some embodiments the electrical stimulation can selectively and/or preferentially modulate afferent neural activity and/or neurological responses to sensory inputs. In such embodiments, the electrical stimulation can impede, inhibit, or dampen afferent neural activity and/or neurological response to sensory inputs. In other such embodiments, the electrical stimulation can promote, enhance or magnify afferent neural activity and/or neurological response to sensory inputs. As another example, in some embodiments the electrical stimulation can selectively and/or preferentially modulate efferent neural activity. In such embodiments, the electrical stimulation can impede, inhibit, or dampen efferent neural activity. In other such embodiments, the electrical stimulation can promote, enhance or magnify efferent neural activity.

Specific details of certain embodiments of the disclosure are described below with reference to methods for modulating one or more target neural populations (e.g., nerves) or sites of a patient, and associated implantable structures for providing the modulation. Although selected embodiments are described below with reference to modulating the dorsal column, dorsal horn, dorsal root, dorsal root entry zone, ventral column, ventral horn, and/or other particular regions of the spinal column, the modulation may in some instances be directed to other neurological structures and/or target neural populations of the spinal cord and/or other neurological tissues. For example, some embodiments may include modulating brain tissue, including the cortex (e.g., motor cortex) and/or deep brain structures. Some embodiments can have configurations, components or procedures different than those described in this section, and other embodiments may eliminate particular components or procedures. A person of ordinary skill in the relevant art, therefore, will understand that the present disclosure may include other embodiments with additional elements, and/or may include other embodiments without several of the features shown and described below with reference to Figures 1-10.

### 3.0 System Characteristics

Figure 1 schematically illustrates a representative patient therapy system 100 for treating a patient's motor, sensory, and/or other functioning, arranged relative to the general anatomy of the patient's spinal column 191. The system 100 can include a signal generator 101 (e.g., an implanted or implantable pulse generator or IPG), which can be implanted subcutaneously within a patient 190 and coupled to one or more signal delivery elements or devices 110. The signal delivery elements or devices 110 can be implanted within the patient 190, at or off the patient's spinal cord midline 189. The signal delivery elements 110 carry features for delivering therapy to the patient 190 after implantation. The signal generator 101 can be connected directly to the signal delivery devices 110, or it can be coupled to the signal delivery devices 110 via a signal link, e.g., a lead extension 102. In some embodiments, the signal delivery devices 110 can include one or more elongated lead(s) or lead body or bodies 111 (identified individually as a first lead 111a and a second lead 111b). As used herein, the terms signal delivery device, signal delivery element, lead, and/or lead body include any of a number of suitable substrates and/or supporting members that carry electrodes/devices for providing therapy signals to the patient 190. For example, the lead or leads 111 can include one or more electrodes or electrical contacts that direct electrical signals into the patient's tissue, e.g., to provide for therapeutic relief. In some embodiments, the signal delivery elements 110 can include structures other than a lead body (e.g., a paddle) that also direct electrical signals and/or other types of signals to the patient 190, e.g., as disclosed in U.S. Patent Application Publication No. 2018/0256892,. For example, paddles can be more suitable for patients with spinal cord injuries that result in scarring or other tissue damage that impedes cylindrical leads.

In some embodiments, one signal delivery device can be implanted on one side of the spinal cord midline 189, and a second signal delivery device can be implanted on the other side of the spinal cord midline 189. For example, the first and second leads 111a, 111b shown in Figure 1 can be positioned just off the spinal cord midline 189 (e.g., about 1 mm offset) in opposing lateral directions so that the two leads 111a, 111b are spaced apart from each other by about 2 mm. In some embodiments, the leads 111 can be implanted at a vertebral level ranging from, for example, about T1 to about T12, or from about T4 to about T12. In some embodiments, one or more signal delivery devices can be implanted at other vertebral levels, e.g., as disclosed in U.S. Patent No. 9,327,121,. In other embodiments, one or more leads 111 can be implanted at or proximate other target neural structures, including brain tissue, peripheral nerves, etc.

The signal generator 101 can transmit signals (e.g., electrical signals) to the signal delivery elements 110 that excite and/or suppress target nerves. The signal generator 101 can include a machine-readable (e.g., computer-readable or controller-readable) medium containing instructions for generating and transmitting suitable therapy signals, such to perform the methods described below with respect to Figure 8. The signal generator 101 and/or other elements of the system 100 can include one or more processor(s) 107, memory unit(s) 108, and/or input/output device(s) 112. Accordingly, the process of providing modulation signals, providing guidance information for positioning the signal delivery devices 110, establishing battery charging and/or discharging parameters, and/or executing other associated functions can be performed by computer-executable instructions contained by, on, or in computer-readable media located at the pulse generator 101 and/or other system components. Further, the pulse generator 101 and/or other system components can include dedicated hardware, firmware, and/or software for executing computer-executable instructions that, when executed, perform any one or more methods, processes, and/or sub-processes described herein. The dedicated hardware, firmware, and/or software also serve as "means for" performing the methods, processes, and/or sub-processes described herein. The signal generator 101 can also include multiple portions, elements, and/or subsystems (e.g., for directing signals in accordance with multiple signal delivery parameters), carried in a single housing, as shown in Figure 1, or in multiple housings. For example, the signal generator can include some components that are implanted (e.g., a circuit that directs signals to the signal delivery device 110), and some that are not (e.g., a power source). The computer-executable instructions can be contained on one or more media that are implanted within the patient and/or positioned external to the patient, depending on the embodiment.

The signal generator 101 can also receive and respond to an input signal received from one or more sources. The input signals can direct or influence the manner in which the therapy, charging, and/or process instructions are selected, executed, updated, and/or otherwise performed. The input signals can be received from one or more sensors (e.g., an input device 112 shown schematically in Figure 1 for purposes of illustration) that are carried by the signal generator 101 and/or distributed outside the signal generator 101 (e.g., at other patient locations) while still communicating with the signal generator 101. The sensors and/or other input devices 112 can provide inputs that depend on or reflect patient state (e.g., patient position, patient posture, and/or patient activity level), and/or inputs that are patient-independent (e.g., time). Still further details are included in U.S. Patent No. 8,355,797,.

In some embodiments, the signal generator 101 and/or signal delivery devices 110 can obtain power to generate the therapy signals from an external power source 103. For example, the external power source 103 can by-pass an implanted signal generator and generate a therapy signal directly at the signal delivery devices 110 (or via signal relay components). The external power source 103 can transmit power to the implanted signal generator 101 and/or directly to the signal delivery devices 110 using electromagnetic induction (e.g., RF signals). For example, the external power source 103 can include an external coil 104 that communicates with a corresponding internal coil (not shown) within the implantable signal generator 101, signal delivery devices 110, and/or a power relay component (not shown). The external power source 103 can transmit power to the implanted signal generator 101 and/or directly to the signal delivery devices 110 in a generally continuous manner such that the system 100 can operate without an internal power source. The external power source 103 can be portable for ease of use.

In some embodiments, the signal generator 101 can obtain the power to generate therapy signals from an internal power source, in addition to or in lieu of the external power source 103. For example, the implanted signal generator 101 can include a non-rechargeable battery or a rechargeable battery to provide such power. When the internal power source includes a rechargeable battery, the external power source 103 can be used to recharge the battery. The external power source 103 can in turn be recharged from a suitable power source (e.g., conventional wall power).

During at least some procedures, an external stimulator or trial modulator 105 can be coupled to the signal delivery elements 110, e.g., during an initial procedure, prior to implanting the signal generator 101. For example, a practitioner (e.g., a physician and/or a company representative) can use the trial modulator 105 to vary the modulation parameters provided to the signal delivery elements 110 in real time, and select optimal or particularly efficacious parameters. These parameters can include the location from which the electrical signals are emitted, as well as the characteristics of the electrical signals provided to the signal delivery devices 110. In some embodiments, input is collected via the external stimulator or trial modulator 105 and can be used by the clinician to help determine what parameters to vary. In a typical process, the practitioner uses a cable assembly 120 to temporarily connect the trial modulator 105 to the signal delivery device 110. The practitioner can test the efficacy of the signal delivery devices 110 in an initial position. The practitioner can then disconnect the cable assembly 120 (e.g., at a connector 122), reposition the signal delivery devices 110, and reapply the electrical signals. This process can be performed iteratively until the practitioner obtains the desired position for the signal delivery devices 110. Optionally, the practitioner can move the partially implanted signal delivery devices 110 without disconnecting the cable assembly 120. Furthermore, in some embodiments, the iterative process of repositioning the signal delivery devices 110 and/or varying the therapy parameters may not be performed.

The signal generator 101, the lead extension 102, the trial modulator 105 and/or the connector 122 can each include a receiving element 109. Accordingly, the receiving elements 109 can be patient implantable elements, or the receiving elements 109 can be integral with an external patient treatment element, device or component (e.g., the trial modulator 105 and/or the connector 122). The receiving elements 109 can be configured to facilitate a simple coupling and decoupling procedure between the signal delivery devices 110, the lead extension 102, the pulse generator 101, the trial modulator 105 and/or the connector 122. The receiving elements 109 can be at least generally similar in structure and function to those described in U.S. Patent Application Publication No. 2011/0071593,.

After the signal delivery elements 110 are implanted, the patient 190 can receive therapy via signals generated by the trial modulator 105, generally for a limited period of time. During this time, the patient wears the cable assembly 120 and the trial modulator 105 outside the body. Assuming the trial therapy is effective or shows the promise of being effective, the practitioner then replaces the trial modulator 105 with the implanted signal generator 101, and programs the signal generator 101 with therapy programs selected based on the experience gained during the trial period. Optionally, the practitioner can also replace the signal delivery elements 110. In still further embodiments, the signal generator 101 can be implanted without first undergoing a trial period. Once the implantable signal generator 101 has been positioned within the patient 190, the therapy programs provided by the signal generator 101 can still be updated remotely via a wireless physician's programmer 117 (e.g., a physician's laptop, a physician's remote or remote device, etc.) and/or a wireless patient programmer 106 (e.g., a patient's laptop, patient's remote or remote device, etc.). Generally, the patient 190 has control over fewer parameters than does the practitioner. For example, the capability of the patient programmer 106 can be limited to starting and/or stopping the signal generator 101, and/or adjusting the signal amplitude within a present amplitude range. The patient programmer 106 can be configured to accept inputs corresponding to pain relief, motor functioning and/or other variables, such as medication use. Accordingly, more generally, embodiments of the present technology include receiving patient feedback, via a sensor, that is indicative of, or otherwise corresponds to, the patient's response to the signal. Feedback includes, but is not limited to, motor, sensory, and verbal feedback. In response to the patient feedback, one or more signal parameters can be adjusted, such as frequency, pulse width, amplitude, or delivery location.

Figure 2 is a cross-sectional illustration of the spinal cord 191 and an adjacent vertebra 195 (based generally on information from Crossman and Neary, "Neuroanatomy," 1995 (published by Churchill Livingstone)), along with multiple leads 111 (shown as leads 111a-111e) implanted at representative locations. For purposes of illustration, multiple leads 111 are shown in Figure 2 implanted in a single patient. In addition, for purposes of illustration, the leads 111 are shown as elongated leads however, leads 111 can be paddle leads. In actual use, any given patient will likely receive fewer than all the leads 111 shown in Figure 2.

The spinal cord 191 is situated within a vertebral foramen 188, between a ventrally located ventral body 196 and a dorsally located transverse process 198 and spinous process 197. Arrows V and D identify the ventral and dorsal directions, respectively. The spinal cord 191 itself is located within the dura mater 199, which also surrounds portions of the nerves exiting the spinal cord 191, including the ventral roots 192, dorsal roots 193, and dorsal root ganglia 194. The dorsal roots 193 enter the spinal cord 191 at the dorsal root entry region 187, and communicate with dorsal horn neurons located at the dorsal horn 186. In some embodiments, the first and second leads 111a, 111b are positioned just off the spinal cord midline 189 (e.g., about 1 mm offset) in opposing lateral directions so that the two leads 111a, 111b are spaced apart from each other by about 2 mm, as discussed above. In some embodiments, a lead or pairs of leads can be positioned at other locations, e.g., toward the outer edge of the dorsal root entry region 187 as shown by a third lead 111c, or at the dorsal root ganglia 194, as shown by a fourth lead 111d, or approximately at the spinal cord midline 189, as shown by a fifth lead 111e.

In some embodiments, the devices and systems of the present technology include features other than those described herein. For example, one lead 111 to six leads 111 can be positioned generally end-to-end at or near the patient's midline M and span vertebral levels from about T4 to about T12. In some embodiments, two, three, or four leads 111 are positioned end-to-end at or near the patient's midline from T4 to T12. In some embodiments, the leads 111 and/or other signal delivery devices can have locations other than those expressly shown herein. For example, one or more signal delivery devices can be positioned at the dorsal side of the spinal cord 191. In addition, the devices and systems of the present technology can include more than one internal stimulator and/or more than one external stimulator that can be configured for wireless stimulation, such as by using electromagnetic waves.

Several aspects of the technology are embodied in computing devices, e.g., programmed/programmable pulse generators, controllers and/or other devices. The computing devices on/in which the described technology can be implemented can include one or more central processing units, memory, input devices (e.g., input ports), output devices (e.g., display devices), storage devices, and network devices (e.g., network interfaces). The memory and storage devices are computer-readable media that can store instructions that implement the technology. In some embodiments, the computer readable media are tangible media. In some embodiments, the data structures and message structures can be stored or transmitted via an intangible data transmission medium, such as a signal on a communications link. Various suitable communications links can be used, including but not limited to a local area network and/or a wide-area network.

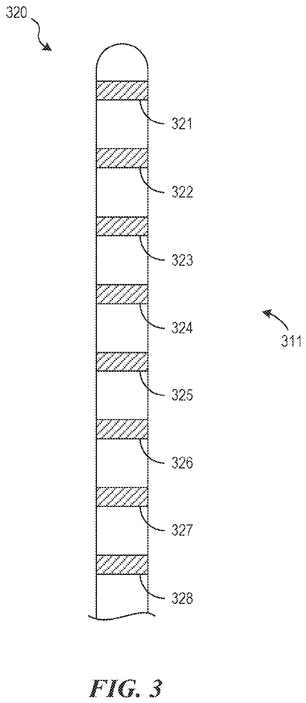

Figure 3 is a partially schematic illustration of a representative lead body 311 that can be used to apply modulation to a patient in accordance with any of the foregoing embodiments. In general, the lead body 311 includes a multitude of electrodes or contacts 320. When the lead body 311 has a circular cross-sectional shape, as shown in Figure 3, the contacts 320 can have a generally ring-type shape and can be spaced apart axially along the length of the lead body 311. In a particular embodiment, the lead body 311 can include eight contacts 320, identified individually as first, second, third . . . eighth contacts 321, 322, 323 . . . 328. In general, one or more of the contacts 320 are used to provide signals, and another one or more of the contacts 320 provide a signal return path. Accordingly, the lead body 311 can be used to deliver monopolar modulation (e.g., if the return contact is spaced apart significantly from the delivery contact), or bipolar modulation (e.g., if the return contact is positioned close to the delivery contact and in particular, at the same target neural population as the delivery contact). In still further embodiments, the pulse generator 101 (Figure 1) can operate as a return contact for monopolar modulation.

### 4.0 Representative Embodiments of the Present Technology

As described in detail below, the present technology includes systems and methods of administering two or more electrical signals with different parameters to a target neural population. The two or more electrical signals can be delivered using different contacts or electrodes located on the same delivery element that is positioned proximate the target neural population. For example, Figure 4A illustrates a representative pattern of administering two different electrical signals using a portion of the lead body 311 shown in Figure 3 and configured in accordance with embodiments of the present technology. In particular, a first set A of the contacts 320, which in the illustrated embodiment includes the first contact 321 and the fourth contact 324, can administer a first electrical signal 402. A second set B of the contacts 320, which in the illustrated embodiment includes the second contact 322 and the third contact 323, can administer a second electrical signal 404. Although the first set A and the second set B of contacts are shown in the illustrated embodiment as each including two contacts, in other embodiments the first set A may include more than two contacts, such as three, four, or more contacts. Likewise, the second set B may include more than two contacts, such as three, four, or more contacts.

In some embodiments, the first electrical signal 402 and the second electrical signal 404 can be delivered concurrently, such that delivery of the first electrical signal 402 and delivery of the second electrical signal 404 temporally overlap (e.g., the first electrical signal 402 and the second electrical signal 404 can be delivered at the same time). In other embodiments, delivery of the first electrical signal 402 and the second electrical signal 404 may be at least partially temporally offset. In yet other embodiments, delivery of the first electrical signal 402 and the second electrical signal 404 do not temporally overlap (e.g., delivery of the first electrical signal 402 is terminated before delivery of the second electrical signal 404, or vice versa).

As set forth the above, the first electrical signal 402 can have different parameters than the second electrical signal 404. In some embodiments, the first electrical signal 402 is a long pulse width signal that has individual pulses with a relatively long pulse width of from about 5 milliseconds to about 2 seconds, or from about 5 milliseconds to about 1 second, or from about 10 milliseconds to about 1 second, or from about 100 milliseconds to about 1 second, or from about 100 milliseconds to about 500 milliseconds. In some embodiments, the first electrical signal 402 may approximate a direct current signal by virtue of the relatively long pulse widths of the individual pulses. In such embodiments, the first electrical signal 402 can behave as a direct current signal even though it is biphasic. Examples of additional long pulse width signals are described in U.S. Patent Publication No. 2021/0228881,. In some embodiments, the first electrical signal 402 is a low-frequency electrical signal having a frequency less than 1.2 kHz. For example, the first electrical signal 402 can have a frequency less than 1 kHz, less than 500 Hz, less than 200 Hz, less than 100 Hz, less than 50 Hz, less than 20 Hz, less than 10 Hz, less than 5 Hz, or less than 1 Hz, such as 0.1 Hz, 0.2 Hz, 0.3 Hz, 0.4 Hz, 0.5 Hz, or the like. In some embodiments, the first electrical signal 402 can be administered at current amplitudes of from 0.1 mA to 20 mA, or 0.5 mA to 10 mA, or 0.5 mA to 7 mA, or 0.5 mA to 5 mA. The first electrical signal 402 can also be administered according to a duty cycle ranging from about 10% to about 100%, such as about 10% to about 50%.

In some embodiments, the second electrical signal 404 is a high frequency electrical signal having a frequency in a frequency range of from about 1.2 kHz to about 500 kHz. For example, the high frequency signal can have a frequency of from about 1.2 kHz to about 100 kHz, or from about 1.5 kHz to about 100 kHz, or from about 2 kHz to about 50 kHz, or from about 3 kHz to about 20 kHz, or from about 3 kHz to about 15 kHz, or from about 5 kHz to about 15 kHz, or from about 3 kHz to about 10 kHz, or 1.5 kHz, 2 kHz, 3 kHz, 4 kHz, 5 kHz, 10 kHz, 15 kHz, 20 kHz, 50 kHz, or 100 kHz. The second electrical signal 404 may have a pulse width of from about 1 microsecond to about 417 microseconds, or from about 10 microseconds to about 333 microseconds, or from about 10 microseconds to about 166 microseconds, or from about 25 microseconds to about 166 microseconds, or from about 20 microseconds to about 100 microseconds, or from about 30 microseconds to about 100 microseconds, or from about 30 microseconds to about 40 microseconds, or from about 10 microseconds to about 50 microseconds, or from about 20 microseconds to about 40 microseconds, or from about 25 microseconds to about 35 microseconds, or from about 30 microseconds to about 35 microseconds, or 30 microseconds. In some embodiments, the second electrical signal 404 can be administered at current amplitudes of from 0.1 mA to 20 mA, or 0.5 mA to 10 mA, or 0.5 mA to 7 mA, or 0.5 mA to 5 mA. The second electrical signal 404 can also be administered according to a duty cycle ranging from about 10% to about 100%, such as about 10% to about 50%.

The first electrical signal 402 delivered via the first set A of contacts 320 generates a first electrical field proximate the lead body 311, and the second electrical signal 404 delivered via the second set B of contacts 320 generates a second electrical field proximate the lead body 311. In the illustrated embodiment, the first electrical field generated by the first electrical signal 402 is larger than and at least partially surrounds the second electrical field generated by the second electrical signal 404. This is because the second electrical signal 404 is delivered via a pair of contacts (the second contact 322 and the third contact 323) that is positioned between the pair of contacts (the first contact 321 and the fourth contact 324) that delivers the first electrical signal 402. In some embodiments, the setup can be reversed, such that the first electrical signal 402 is delivered using the second set B of contacts and the second electrical signal 404 is delivered using the first set A of contacts.

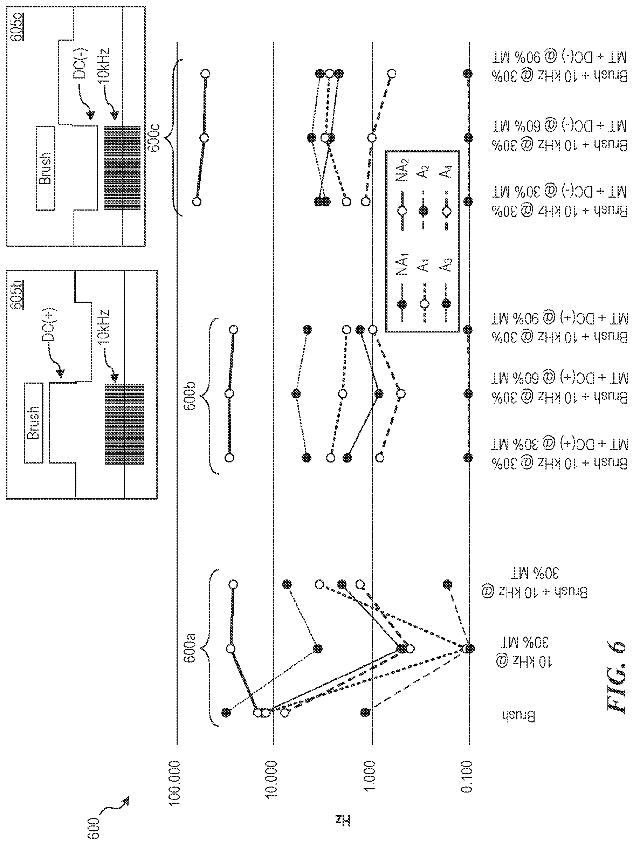

Because the first electrical signal 402 and the second electrical signal 404 are biphasic, the direction of current flow through patient tissues reverses depending on the phase of the electrical signal. However, the contacts 320 can nevertheless be selectively programmed as either a cathode or an anode for a leading phase of a biphasic electrical signal. Thus, the initial direction of current flow for a given electrical signal can be programmed. As described in greater detail below, the direction of flow for electrical signals that approximate a direct current signal may impact the modulatory effect the electrical signal has on certain neurons. Accordingly, as described in detail below with reference to Figures 6-9, the direction of current flow for signals that mimic direct current signals can be selected based on a desired modulatory effect.

As set forth above, delivery of the second electrical signal 404 can at least partially temporally overlap with delivery of the first electrical signal 402, such that the first and second electrical fields are simultaneously generated in patient tissue. In such embodiments, the timing of the second electrical signal 420 relative to the first electrical signal 402 can be varied. For example, although the second electrical signal 404 is shown as being administered during a leading phase of the first electrical signal 402 and as not being administered during the charge balancing phase of the first electrical signal 402, in other embodiments the second electrical signal 404 is delivered during both the leading phase and the charge balancing phase of the first electrical signal 420, or only during the charge balancing phase of the first electrical signal 402. In some embodiments, the second electrical signal 404 may be delivered in a continuous or at least substantially continuous manner (e.g., applied at a duty cycle of 50% or more).

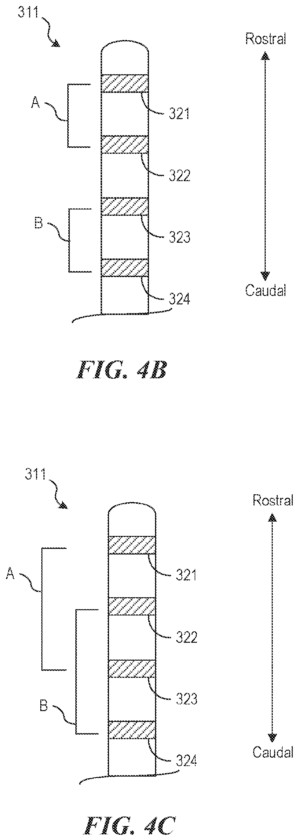

The contacts 320 included in a given set can also be varied to change the shape of the electrical field generated by the first electrical signal 402 and/or the second electrical signal 404. For example, Figure 4B illustrates a second representative pattern of administering multiple electrical signals using the same portion of the lead body 311 shown in Figure 4A. However, relative to the pattern shown in Figure 4B, the first set A of electrodes that delivers the first electrical signal 402 includes the first contact 321 and the second contact 322, and the second set B of electrodes that delivers the second electrical signal 404 includes the third contact 323 and the fourth contact 324. As a result, the first electrical field generated by the first electrical signal 402 does not "surround" the second electrical field generated by the second electrical signal 404. Rather, the first electrical field and the second electrical field have a "side-by-side" configuration, and may or may not partially overlap. Figure 4C illustrates a third representative pattern of administering multiple electrical signals using the same portion of the lead body 311 shown in Figure 4A. Relative to the patterns shown in Figures 4A and 4B, the first set A and the second set B of contacts are staggered, such that the first set A that delivers the first electrical signal 402 includes the first contact 321 and the third contact 323, and the second set B that delivers the second electrical signal 404 includes the second contact 322 and the fourth contact 324. Accordingly, one skilled in the art will appreciate that any combination of two or more electrodes on the lead body 311 may comprise the first set A, and any combination of two or more different electrodes on the lead body 311 (e.g., electrodes not included in the first set A) may comprise the second set B. In some embodiments, the selection of electrodes for the first set A and the second set B can be based at least in part on the desired shape of the electrical field to be generated by the first electrical signal 402 and/or the second electrical signal 404.

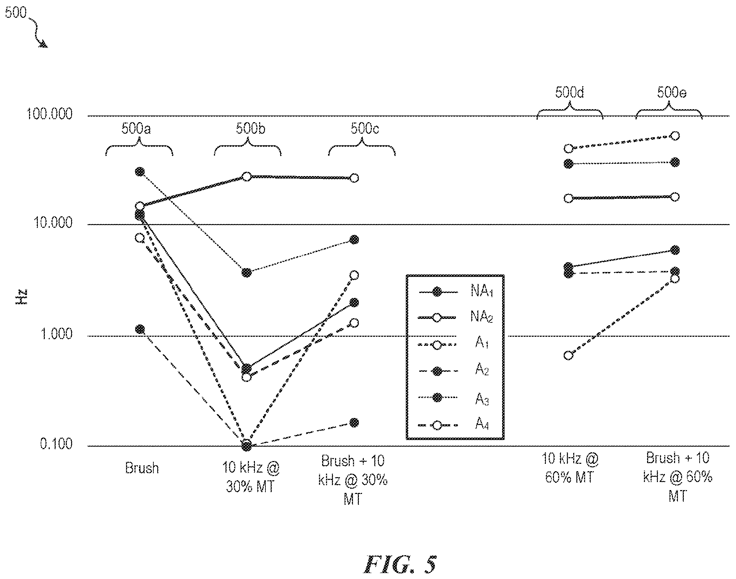

Electrical stimulation in accordance with embodiments of the present technology can have a variety of effects on the neural activity of a target neural population. Figure 5 illustrates a line graph 500 showing activity of six neurons under various conditions/in response to various stimuli in accordance with embodiments of the present technology. In particular, the x-axis shows the various conditions during which neural activity was measured, and the y-axis shows the firing rate for each neuron under each condition in Hertz (Hz). The neural activity was measured for six neurons: two non-adapting neurons NA₁ and NA₂, and four adapting neurons A₁₋A₄. The neural activity of each of the six neurons was measured under five conditions: (1) activity during a brush stimulus (data set 500a), (2) activity when stimulated by a 10 kHz electrical signal applied at 30% of motor threshold in the absence of the brush stimulus (data set 500b), (3) activity during a brush stimulus while also being stimulated by a 10 kHz electrical signal applied at 30% of motor threshold (data set 500c), (4) activity when stimulated by a 10 kHz electrical signal applied at 60% of motor threshold in the absence of the brush stimulus (data set 500d), and (5) activity during a brush stimulus while also being stimulated by a 10 kHz electrical stimulation applied at 60% of motor threshold (data set 500e).

To obtain the data illustrated in Figure 5, an operator manually applied the brush stimulus. The 10 kHz electrical signal was applied as a bi-phasic electrical signal generally similar to the second electrical signal described with reference to Figures 4A-4C. For the data sets that tested the effect of 10kHz electrical signals on the neural response to the brush stimulus (data sets 500b-500e), delivery of the 10 kHz signal was synchronized with delivery of the brush stimulus.

Five of the six neurons (non-adapting neuron NA₁ and adapting neurons A₁-A₄) demonstrated higher activity in response to the brush stimulus than in response to the 10 kHz electrical signal at 30% motor threshold, as shown by the data set 500a and the data set 500b. The only neuron to demonstrate higher activity in response to the 10 kHz electrical signal at 30% motor threshold than in response to the brush stimulus was non-adapting neuron NA₂. Despite this, and without being bound by theory, it is expected that for a larger population of neurons, neural activity in response to the 10 kHz electrical signal at 30% motor threshold would generally be lower than neural activity in response to the brush stimulus.

For each of the five neurons that demonstrated higher activity to the brush stimulus than to the 10 kHz electrical signal, the 10 kHz electrical signal at 30% motor threshold reduced their activity in response to the brush stimulus when the brush stimulus and 10 kHz electrical signal were applied concurrently, as shown in the data set 500c. The 10 kHz electrical signal applied at 30% of motor threshold therefore at least partially suppressed the response of these neurons to the brush stimulus.

The activity of the neurons in response to the 10 kHz electrical signal applied at 60% of motor threshold was greater than the activity of the neurons in response to the 10 kHz electrical signal applied at 30% of motor threshold, as shown in the data set 500d. Four neurons (non-adapting neuron NA₂ and adapting neurons A₂-A₄) demonstrated greater activity in response to the 10 kHz electrical signal applied at 60% of motor threshold than in response to the brush stimulus alone. Two neurons (non-adapting neuron NA₁ and adapting neuron A₁) demonstrated less activity in response to the 10 kHz electrical signal applied at 60% of motor threshold than in response to the brush stimulus alone. For the four neurons that demonstrated greater activity to the 10 kHz electrical signal applied at 60% of motor threshold, the 10 kHz electrical signal at 60% motor threshold did not reduce their response to the brush stimulus relative to their response to the brush stimulus in the absence of electrical stimulation, as shown in the data set 500e. Rather, the 10 kHz electrical signal applied at 60% of motor threshold magnified the response of these neurons to the brush stimulus. This is in contrast to the effect the 10 kHz electrical signal had when applied at 30% of motor threshold.

Figure 6 is a line graph 600 illustrating the activity of the six neurons described with reference to Figure 5 in response to additional combinations of stimuli in accordance with embodiments of the present technology. The x-axis shows the various conditions during which neural activity was measured, and the y-axis shows the firing rate for each neuron under each condition. The stimuli used to obtain the data shown in Figure 6 included a brush stimulus, a high frequency electrical signal, and a long pulse width electrical signal. The brush stimulus was the same as the brush stimulus described with reference to Figure 5. The high frequency electrical signal was applied at a frequency of 10 kHz and at 30% of the motor threshold (e.g., the same parameters used for the tests shown in Figure 5). The long pulse width electrical signal had a first phase (e.g., a cathodic phase or an anodic phase) having a pulse width of about 1 second followed by a second phase (e.g., the other of the cathodic phase or the anodic phase) having a pulse width of about 1 second for charge balancing. Accordingly, the long pulse width electrical signal is labeled in Figure 6 as a direct current "DC" because it had a relatively long pulse width that approximated a direct current signal. The high frequency and long pulse width electrical signals were applied using the pattern of administration described with reference to Figure 4A, with the long pulse width electrical signal being delivered via a first pair of contacts (e.g., the first contact 321 and the fourth contact 324 of the lead 311; Figures 3 and 4A) surrounding a second pair of contacts (e.g., the second contact 322 and the third contact 323 of the lead 311) delivering the high frequency electrical signal.

The graph 600 includes three data sets: a first data set 600a, a second data set 600b, and a third data set 600c. The first data set 600a illustrates the same data as the first three data sets 500a-c shown in the graph 500 of Figure 5: the neural activity of the six neurons (1) during a brush stimulus in the absence of any electrical stimulation, (2) during 10 kHz electrical stimulation at 30% motor threshold, and (3) during a brush stimulus while also being stimulated by the 10 kHz electrical signal at 30% motor threshold.

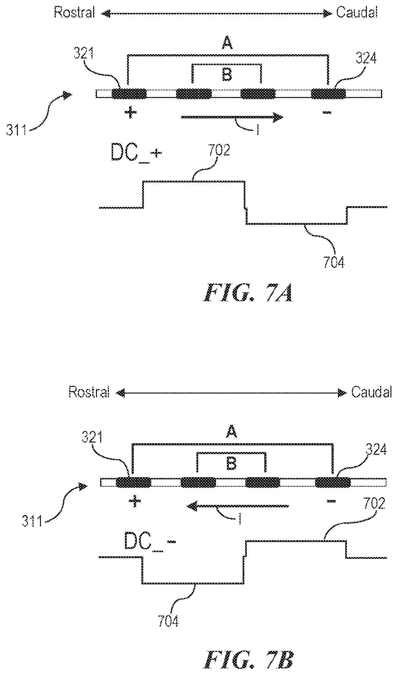

The second data set 600b illustrates the activity of each of the six neurons in response to a brush stimulus during application of both the high frequency electrical signal at 30% motor threshold (i.e., the 10 kHz electrical signal) and the long pulse width electrical signal at 30% motor threshold, 60% motor threshold, and 90% motor threshold, respectively. Box 605b schematically illustrates the stimuli applied to obtain the data shown in the data set 600b, and Figure 7A illustrates additional details of the pattern used to administer the long pulse width electrical signal DC(+). As shown in Figure 7A, the long pulse width electrical signal DC(+) was applied with an anodic phase 702 as the leading phase and a cathodic phase 704 as the charge balancing phase, relative to the first contact 321. As a result, electrical current I flowed in a rostral to caudal direction (i.e., from the first contact 321 toward the fourth contact 324) during the leading (i.e., anodic) phase. Of course, the flow of electrical current I (and the polarity of the first contact 321 and the fourth contact 324) was reversed during the charge balancing phase (i.e., the cathodic phase 704). However, due to the relatively long pulse width used for the anodic phase 702, the application of the long pulse width signal DC(+) approximated a direct current signal with current flowing in the rostral to caudal direction for at least the duration of the leading (i.e., anodic) phase.

Returning to Figure 6, the third data set 600c illustrates the activity of each of the six neurons in response to the same conditions as shown in the second data set 600b, except that the polarity of the leading phase of the long pulse width electrical signal was opposite that from the data set 600b. Box 605c schematically illustrates the stimuli applied to obtain the data shown in the data set 600c, and Figure 7B illustrates additional details of the pattern used to administer the long pulse width electrical signal DC(-). As shown in Figure 7B, the long pulse width electrical signal DC(-) was applied with the cathodic phase 704 as the leading phase and the anodic phase 702 as the charge balancing phase, relative to the first contact 321. As a result, electrical current I flowed in a caudal to rostral direction (i.e., from the fourth contact 324 toward the first contact 321) during the leading (i.e., cathodic) phase. This is the opposite direction that current flowed during the leading phase of the long pulse width electrical signal DC(+) described with respect to data set 600b and Figure 7A. Of course, the flow of electrical current I (and the polarity of the first contact 321 and the fourth contact 324) was reversed during the charge balancing phase (i.e., the anodic phase 702). However, due to the relatively long pulse width used for the cathodic phase 704, the application of the long pulse width electrical signal DC(-) approximated a direct current signal with current flowing in the caudal to rostral direction for at least the duration of the leading (i.e., cathodic) phase.

For adapting neurons A₁-A₄, both directions of current flow for the long pulse width electrical signal during the leading phase (i.e., both DC(+) and DC(-)) decreased the activity of these neurons when applied with the brush stimulus and the high frequency stimulus, relative to the activity of these neurons to the brush stimulus and the high frequency stimulus in the absence of the long pulse width stimulus (e.g., comparing the second data set 600b and the third data set 600b to the first data set 600a). Accordingly, without being bound by theory, it is expected that the direction of current flow does not affect (e.g., substantially affect) the impact the long pulse width electrical signal has on adapting neurons. In contrast, for non-adapting neurons NA₁ and NA₂, the long pulse width electrical signal DC(+) with an anodic leading phase during which current flowed in a rostral to caudal direction did not substantially affect or at least partially reduced the activity of these neurons (as shown in the second data set 600b), whereas the long pulse width electrical signal DC(-) with a cathodic leading phase during which current flowed in a caudal to rostral direction increased the activity of these neurons (as shown in third data set 600c). Accordingly, without being bound by theory, it is expected that the direction of current flow for a long pulse width electrical signal may affect the impact electrical stimulation has on non-adapting neurons. Therefore, in some embodiments, the direction of current flow for the leading phase of the long pulse width electrical signal can be selected based on a desired effect on a target neural population. For example, an electrical signal with current flowing in the caudal to rostral direction (e.g., during the leading phase of the electrical signal) can be applied to increase activity of non-adapting neurons, and an electrical signal with current flowing in the rostral to caudal direction (e.g., during the leading phase of the electrical signal) can be applied to decrease activity of non-adapting neurons.

The present technology can therefore include selectively and/or preferentially enhancing or suppressing peripheral sensory stimuli and/or afferent neural activity. For example, a neural response to sensory stimuli (e.g., afferent neural activity) can be suppressed by administering a long pulse width electrical signal that causes current to flow in a first direction. Likewise, a neural response to sensory stimuli (e.g., afferent neural activity) can be enhanced by administering a long pulse width electrical signal that causes current to flow in a second direction (e.g., opposite the first direction). Although described as a first direction and a second direction, one skilled in the art will appreciate that the direction of flow for a biphasic electrical signal will reverse depending on the phase of the electrical signal. Accordingly, the use of the term "first direction" and "second direction" in the context of current flow for biphasic signals refers to the direction of current flow during a leading (e.g., initial) phase of the electrical signal, and does not necessarily mean that the electrical signal demonstrates the first or second direction of current flow throughout its entire duration. For example, for a biphasic electrical signal having a "first direction" of current flow, the electrical signal has current flow in the first direction during a leading phase of the electrical signal, and may have current flow in the second direction during a charge balancing phase of the electrical signal. Likewise, for a biphasic electrical signal having a "second direction" of current flow, the electrical signal has current flow in the second direction during the leading phase of the electrical signal, and may have current flow in the first direction during the charge balancing phase of the electrical signal. In some embodiments, the first direction of current flow is rostral to caudal, and the second direction of current flow is caudal to rostral. In other embodiments, the first direction of current flow is caudal to rostral, and the second direction of current flow is rostral to caudal. Therefore, a user can program a signal generator to administer an electrical signal with a specific direction of current flow depending on the desired impact in the patient.

In some embodiments, a similar pattern is expected for tuning efferent neural activity. In such embodiments, a first direction of current flow may enhance efferent neural activity and a second direction of current flow may suppress efferent neural activity. For example, an electrical signal with current flowing in a rostral to caudal direction may enhance efferent neural activity, and an electrical signal with current flowing in a caudal to rostral direction may inhibit efferent neural activity. In another example, the effects can be reversed, such that the electrical signal with current flowing in the caudal to rostral direction enhances efferent neural activity, and the electrical signal with current flowing in the rostral to caudal direction inhibits neural activity.

Without being bound by theory, one potential mechanism of action explaining the foregoing data is that the long pulse width signals have a different modulatory impact than the 10 kHz electrical signal. For example, the 10 kHz electrical signal may preferentially modulate neurons at a first location, such as nerve endings (e.g., nerve synapses) or nerve fibers (e.g., the nerve axon), whereas the long pulse width signal may preferentially modulate neurons at a second location, such as the other of nerve endings or nerve fibers. Similarly, the 10 kHz electrical signal and/or the long pulse width electrical signal may preferentially modulate a particular type of neuron, such as interneurons. In some embodiments, the long pulse width electrical signals may change the membrane voltage for a particular neuron and hold the membrane voltage at the changed level for a duration of the individual pulses of the long pulse width signals. Of course, the foregoing effects may be the result of other mechanisms, or combinations of mechanisms. Thus, the present technology is not limited by the foregoing description of mechanism of action, except where expressly noted.

### 5.0 Methods for Suppressing or Enhancing Neural Activity

The present technology further includes methods for treating a patient, such as by preferentially and/or selectively suppressing and/or enhancing afferent neural activity and/or efferent neural activity at a target neural population. For example, Figure 8 is a block diagram illustrating a method 800 for treating a patient in accordance with embodiments of the present technology. Some or all of the operations in the method 800 can be performed by a processor executing instructions stored on one or more elements of a patient treatment system.

The method 800 can begin at block 802 by delivering a first electrical signal to a target neural population via a first set of electrodes of a signal delivery device. The first electrical signal can be a long pulse width electrical signal, and can be the same as or generally similar to the first electrical signal described with reference to Figures 4A-4C.. In some embodiments, the first electrical signal includes individual pulses having relatively long pulse widths (e.g., between about 5 milliseconds and about 2 seconds) such that the individual pulses of the first electrical signal approximate a direct current signal (e.g., even in embodiments in which the first electrical signal is bi-phasic). The first set of electrodes can include two or more electrodes, as previously described.

The method 800 can continue in block 804 by delivering a second electrical signal different than the first electrical signal to the target neural population via a second set of electrodes of the signal delivery device. The second electrical signal can be a high frequency electrical signal, and can be the same as or generally similar to the second electrical signal described with reference to Figures 4A-4C. The second set of electrodes can include two or more electrodes. The two or more electrodes used to deliver the first electrical signal can be different than the two or more electrodes used to deliver the second electrical signal, as described with reference to Figures 4A-4C. In some embodiments, the operations of block 802 and block 804 are performed simultaneously, such that the first electrical signal and the second electrical signal are administered at the same time. In some embodiments, the operations of block 802 and block 804 are at least partially offset, such that delivery of the first electrical signal and the second electrical signal are at least partially temporally offset.

In some embodiments, the first electrical signal and/or the second electrical signal can be tuned to preferentially suppress or enhance afferent neural activity and/or the activity of the target neural population in response to a sensory stimulus. For example, the first electrical signal can be applied with a first direction of current flow to suppress afferent neural activity (and/or suppress the activity of the target neural population) and a second direction of current flow opposite the first direction of current flow to enhance afferent neural activity (and/or enhance the activity of the target neural population in response to a sensory stimulus). Although described as a first direction and a second direction, one skilled in the art will appreciate that the direction of flow for a biphasic electrical signal will reverse depending on the phase of the electrical signal. Accordingly, the use of the term "first direction" and "second direction" in the context of current flow for biphasic signals refers to the direction of current flow during a leading (e.g., initial) phase of the electrical signal, and does not necessarily mean that the electrical signal demonstrates the first or second direction of current flow throughout its entire duration. For example, for a biphasic electrical signal having a "first direction" of current flow, the electrical signal has current flow in the first direction during a leading phase of the electrical signal, and may have current flow in the second direction during a charge balancing phase of the electrical signal. Likewise, for a biphasic electrical signal having a "second direction" of current flow, the electrical signal has current flow in the second direction during the leading phase of the electrical signal, and may have current flow in the first direction during the charge balancing phase of the electrical signal. In some embodiments, the first direction of current flow is rostral to caudal, and the second direction of current flow is caudal to rostral. In other embodiments, the first direction of current flow is caudal to rostral, and the second direction of current flow is rostral to caudal.

In some embodiments, the first electrical signal and/or the second electrical signal can be tuned to preferentially suppress or enhance efferent neural activity. For example, the first electrical signal can be applied with a first direction of current flow to suppress efferent neural activity at the target neural population, and with a second direction of current flow opposite the first direction of current flow to enhance efferent neural activity at the target neural population. As set forth above, the use of the term "first direction" and "second direction" in the context of current flow for biphasic signals refers to the direction of current flow during a leading (e.g., initial) phase of the electrical signal, and does not necessarily mean that the electrical signal demonstrates the first or second direction of current flow throughout its entire duration. In some embodiments, the first direction of current flow is caudal to rostral, and the second direction of current flow is rostral to caudal. In other embodiments, the first direction of current flow is rostral to caudal, and the second direction of current flow is caudal to rostral.

Tuning the electrical signals to preferentially suppress or enhance afferent neural activity and/or efferent neural activity can also be described based on the polarity of a contact used to administer the electrical signal. For example, the first electrical signal can be applied with a first polarity (with reference to a specific contact) to suppress afferent neural activity and/or efferent neural activity, and a second polarity (with reference to the specific contact) to enhance afferent neural activity and/or efferent neural activity. Although described as a "first polarity" and a "second polarity," one skilled in the art will appreciate that the polarity (with reference to a specific contact) of a biphasic electrical signal will change depending on the phase of the electrical signal. Accordingly, the use of the term "first polarity" and "second polarity" refers to the polarity (with reference to the specific contact) during the leading (e.g., initial) phase of the electrical signal, and does not necessarily mean that the electrical signal demonstrates the first or second polarity throughout its entire duration. For example, an electrical signal having the first polarity (with reference to a specific contact) can include bi-phasic pulses that include a leading phase with the first polarity and a charge balancing phase following the leading phase with the second polarity. Likewise, electrical signals having the second polarity can include bi-phasic pulses that include a leading phase with the second polarity and a charge balancing phase following the leading phase with the first polarity.

As provided above, the present technology also includes methods of programming a patient treatment system to perform some or all of the method 800. For example, the present technology includes a patient treatment system including a signal generator and a signal delivery element. The signal delivery element can be configured to be implanted proximate a target neural population. The signal generator can be programmed with instructions for generating the high frequency electrical signal and the long pulse width electrical signal, and directing the high frequency electrical signal and the long pulse width electrical signal to the signal delivery device.

### 6.0 Representative Clinical Applications

Without being bound by theory, embodiments of the present technology are expected to provide certain clinical benefits that may not be attainable using conventional therapeutic approaches. For example, the present technology can be utilized to selectively enhance or suppress afferent neural activity and/or efferent neural activity. In some embodiments, the present technology can be applied to other neural structures beyond the spinal cord. For example, the present technology can be utilized for modulating neurons located in the patient's brain (e.g., deep brain structures) or the patient's peripheral nervous system.

In a first representative example, the electrical stimulation described in Sections 4.0 and 5.0 is applied to a patient's spinal cord, such as to treat pain or another indication. Without being bound by theory, it is expected that the present technology may provide a targeted spinal cord stimulation therapy. For example, the electrical stimulation described herein can be tuned to selectively suppress afferent neurons while having a minimal impact on efferent neurons, or vice versa.

In a second representative example, the electrical stimulation described in Sections 4.0 and 5.0 is applied to one or more peripheral nerves that directly innervate muscle tissue. The electrical stimulation can be selected to specifically induce a motor response in the muscle tissue while suppressing or at least minimizing any accompanying sensory response. Without being bound by theory, this may reduce the precision with which electrical leads must be positioned for peripheral stimulation because, even if the stimulation lead generates an electrical field covering motor neurons and sensory neurons, the electrical stimulation can be "tuned" using the techniques described herein to preferentially activate one type of neuron relative to the other. However, the electrical stimulation described herein may also be applied in embodiments in which the lead is positioned proximate only one type of neuron (e.g., motor neurons or sensory neurons). Representative peripheral nerve targets include the dorsal ramus nerve, which innervates the multifidus muscles that stabilize the lumbar spine. In other embodiments, other peripheral nerves that innervate muscular tissue can be stimulated.

In a third representative example, the electrical stimulation described in Sections 4.0 and 5.0 is applied to the vagus nerve to treat depression, epilepsy, pain, and/or other indications that can be treated by vagus nerve stimulation. The vagus nerve is a large nerve bundle that includes both sensory and motor fibers. Without being bound by theory, it is expected that the electrical stimulation described herein can provide a targeted vagus nerve stimulation therapy. For example, the electrical stimulation described herein can be tuned to selectively enhance sensory fiber activity of the vagus nerve while having a minimal impact on motor fiber activity of the vagus nerve, or vice versa. As a result, the present therapy may induce fewer side effects than conventional vagus nerve stimulation.

The foregoing representative clinical applications are provided by way of example only. From the disclosure herein, one skilled in the art will appreciate that the electrical stimulation described above in Sections 4.0 and 5.0 can be applied to other targets and to treat indications beyond those expressly discussed herein.

### 8.0 Conclusion

From the foregoing, it will be appreciated that specific embodiments of the disclosed technology have been described herein for purposes of illustration, but that various modifications may be made without deviating from the technology. For example, therapy signals described herein can be delivered at combinations of parameter values within the foregoing ranges at values that are not expressly disclosed herein. Certain aspects of the technology described in the context of particular embodiments may be combined or eliminated in other embodiments. For example, the therapy signal can be monophasic with a passive charge elimination phase. In some embodiments, the foregoing techniques can be used to address patient deficits than pain.

The use of "and/or" in reference to a list of two or more items is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded.

Where a range of values is provided, it is understood that each intervening value, to the tenth of the unit of the lower limit unless the context clearly dictates otherwise, to between the upper and lower limits of that range is also specifically disclosed. Each smaller range between any stated value or intervening value in a stated range and any other stated or intervening value in that stated range is encompassed within the disclosure. The upper and lower limits of these smaller ranges may independently be included or excluded in the range, and each range where either, neither or both limits are included in the smaller ranges is also encompassed within the disclosure, subject to any specifically excluded limit in the stated range. Where the stated range includes one or both of the limits, ranges excluding either or both of those included limits are also included in the disclosure.

## Claims

1. A patient treatment system (100), comprising:
- a signal delivery device (110) having a plurality of electrodes (320) and configured to be implanted proximate a target neural population of a patient (190); and
- a signal generator (101) having a computer readable storage medium with instructions that, when executed, cause the signal generator (101) to:
✔ deliver a first electrical signal (402) to a target neural population of the patient (190) via a first set of electrodes (A) of the signal delivery device (110), the first electrical signal (402) having individual pulses having a pulse width within a pulse width range of from 5 ±10% milliseconds to 2 ±10% seconds, and
✔ deliver a second electrical signal (404) to the target neural population via a second set of electrodes (B) of the signal delivery device (110), the second electrical signal (404) having a frequency within a frequency range of from 1.2 ±10% kHz to 100 ±10% kHz,
✔ wherein the second set of electrodes (B) is different than the first set of electrodes (A), and wherein delivery of the first electrical signal (402) at least partially temporally overlaps with delivery of the second electrical signal (404);
wherein the first electrical signal (402) includes bi-phasic pulses with a leading phase having a pulse width in the pulse width range of from 5 ±10% milliseconds to 2 ±10% seconds, followed by a charge balancing phase and wherein the instructions, when executed, cause the signal generator (101) to deliver the leading phase such that current flows in a first direction along the patient's spinal cord (191) during the leading phase, wherein the first direction is associated with enhancing neural activity of the target neural population.

2. The system of claim 1 wherein the first electrical signal (402) has pulse width range from 10 ±10% milliseconds to 1 ±10% second.

3. The system of claim 1 wherein the first direction is caudal to rostral.

4. The system of claim 1 wherein the instructions, when executed, cause the signal generator (101) to deliver the leading phase such that current flows in a second direction along the patient's spinal cord during the leading phase, wherein the second direction is associated with suppressing neural activity of the target neural population.

5. The system of claim 4 wherein the second direction is rostral to caudal.

6. The system of claim 1 wherein the target neural population includes non-adapting neurons.

7. The system of claim 1 wherein: the first set of electrodes (A) includes a first electrode (321) and a second electrode (324); the second set of electrodes (B) includes a third electrode (322) and a fourth electrode (323); and the third electrode (322) and the fourth electrode (323) are positioned between the first electrode (321) and the second electrode (324) along the signal delivery device (110).

8. The system of claim 1 wherein the target neural population includes neurons in a spinal cord of the patient (190), and wherein the implanted signal delivery device (110) is configured to be positioned proximate the spinal cord (191) of the patient (190).

9. The system of claim 1 wherein the target neural population includes one or more peripheral nerves of the patient (190), and wherein the implanted signal delivery device (110) is configured to be positioned proximate the one or more peripheral nerves.

10. The system of claim 9 wherein the one or more peripheral nerves include one or more neurons that directly innervate muscle tissue.

11. The system of claim 9 wherein the one or more peripheral nerves include the vagus nerve.

12. The system of claim 7 wherein the third electrode (322) is positioned between the first electrode (321) and the second electrode(324), and the second electrode (324) is positioned between the third electrode (322) and the fourth electrode (323).

## Patentansprüche

1. Patientenbehandlungssystem (100), umfassend:
- eine Signalabgabevorrichtung (110), die eine Mehrzahl von Elektroden (320) aufweist und eingerichtet ist, um in der Nähe einer neuronalen Zielpopulation eines Patienten (190) implantiert zu werden; und
- einen Signalgenerator (101) mit einem computerlesbaren Speichermedium mit Anweisungen, die bei Ausführung den Signalgenerator (101) veranlassen, um:
- ein erstes elektrisches Signal (402) an eine neuronale Zielpopulation des Patienten (190) über einen ersten Elektrodensatz (A) der Signalabgabevorrichtung (110) abzugeben, wobei das erste elektrische Signal (402) einzelne Impulse mit einer Impulsbreite innerhalb eines Impulsbreitenbereichs von 5 ±10 % Millisekunden bis 2 ± 10 % Sekunden aufweist, und
- ein zweites elektrisches Signal (404) an die neuronale Zielpopulation über einen zweiten Elektrodensatz (B) der Signalabgabevorrichtung (110) abzugeben, wobei das zweite elektrische Signal (404) eine Frequenz in einem Frequenzbereich von 1,2 ± 10 % kHz bis 100 ±10 % kHz aufweist,
- wobei sich der zweite Elektrodensatz (B) vom ersten Elektrodensatz (A) unterscheidet und wobei sich eine Abgabe des ersten elektrischen Signals (402) zumindest teilweise zeitlich mit der Abgabe des zweiten elektrischen Signals (404) überlappt;
- wobei das erste elektrische Signal (402) zweiphasige Impulse mit einer voreilenden Phase mit einer Impulsbreite im Impulsbreitenbereich von 5 ± 10 % Millisekunden bis 2 ± 10 % Sekunden umfasst, gefolgt von einer Ladungsausgleichsphase, und wobei die Anweisungen bei Ausführung den Signalgenerator (101) veranlassen, die voreilende Phase abzugeben, so dass Strom in eine erste Richtung entlang des Rückenmarks (191) des Patienten während der voreilenden Phase fließt, wobei die erste Richtung mit der Steigerung der neuronalen Aktivität der neuronalen Zielpopulation verbunden ist.

2. System nach Anspruch 1, wobei das erste elektrische Signal (402) einen Impulsbreitenbereich von 10±10% Millisekunden bis 1±10% Sekunde aufweist.

3. System nach Anspruch 1, wobei die erste Richtung von kaudal nach rostral verläuft.

4. System nach Anspruch 1, wobei die Anweisungen bei Ausführung den Signalgenerator (101) veranlassen, die voreilende Phase abzugeben, so dass Strom in eine zweite Richtung entlang des Rückenmarks des Patienten während der voreilenden Phase fließt, wobei die zweite Richtung mit einer Unterdrückung der neuronalen Aktivität der neuronalen Zielpopulation verbunden ist.

5. System nach Anspruch 4, wobei die zweite Richtung von rostral nach kaudal verläuft.

6. System nach Anspruch 1, wobei die neuronale Zielpopulation nicht adaptierende Neuronen umfasst.

7. System nach Anspruch 1, wobei: der erste Elektrodensatz (A) eine erste Elektrode (321) und eine zweite Elektrode (324) umfasst; der zweite Elektrodensatz (B) eine dritte Elektrode (322) und eine vierte Elektrode (323) umfasst; und die dritte Elektrode (322) und die vierte Elektrode (323) zwischen der ersten Elektrode (321) und der zweiten Elektrode (324) entlang der Signalabgabevorrichtung (110) positioniert sind.

8. System nach Anspruch 1, wobei die neuronale Zielpopulation Neuronen in einem Rückenmark des Patienten (190) umfasst, und wobei die implantierte Signalabgabevorrichtung (110) eingerichtet ist, um in der Nähe des Rückenmarks (191) des Patienten (190) positioniert zu werden.

9. System nach Anspruch 1, wobei die neuronale Zielpopulation einen oder mehrere periphere Nerven des Patienten (190) umfasst und wobei die implantierte Signalabgabevorrichtung (110) eingerichtet ist, um in der Nähe eines oder mehrerer peripherer Nerven positioniert zu werden.

10. System nach Anspruch 9, wobei ein oder mehrere periphere Nerven ein oder mehrere Neuronen umfassen, die Muskelgewebe direkt innervieren.

11. System nach Anspruch 9, wobei ein oder mehrere periphere Nerven den Vagusnerv umfassen.

12. System nach Anspruch 7, wobei die dritte Elektrode (322) zwischen der ersten Elektrode (321) und der zweiten Elektrode (324) positioniert ist und die zweite Elektrode (324) zwischen der dritten Elektrode (322) und der vierten Elektrode (323) positioniert ist.

## Revendications

1. Système de traitement d'un patient (100), comprenant :
- un dispositif de délivrance de signaux (110) présentant une pluralité d'électrodes (320) et conçu pour être implanté à proximité d'une population neuronale cible d'un patient (190) ; et
- un générateur de signaux (101) présentant un support de stockage lisible par ordinateur contenant des instructions qui, lorsqu'elles sont exécutées, amènent le générateur de signaux (101) à :
✔ délivrer un premier signal électrique (402) à une population neuronale cible du patient (190) par l'intermédiaire d'un premier ensemble d'électrodes (A) du dispositif de délivrance de signaux (110), le premier signal électrique (402) présentant des impulsions individuelles ayant une largeur d'impulsion dans une plage de largeurs d'impulsion allant de 5 ± 10 % millisecondes à 2 ± 10 % secondes, et
✔ délivrer un second signal électrique (404) à la population neuronale cible par l'intermédiaire d'un second ensemble d'électrodes (B) du dispositif de délivrance de signaux (110), le second signal électrique (404) présentant une fréquence dans une plage de fréquences allant de 1,2 ± 10 % kHz à 100 ± 10 % kHz,
✔ dans lequel le second ensemble d'électrodes (B) est différent du premier ensemble d'électrodes (A), et dans lequel la délivrance du premier signal électrique (402) chevauche au moins partiellement, dans le temps, la délivrance du second signal électrique (404) ;
dans lequel le premier signal électrique (402) comprend des impulsions biphasiques avec une phase initiale ayant une largeur d'impulsion dans une plage de largeurs d'impulsion allant de 5 ± 10 % millisecondes à 2 ± 10 % secondes, suivie d'une phase d'équilibrage de charge, et dans lequel les instructions, lorsqu'elles sont exécutées, amènent le générateur de signaux (101) à délivrer la phase initiale de telle sorte qu'un courant circule dans une première direction le long de la moelle épinière (191) du patient pendant la phase initiale, la première direction étant associée à une augmentation de l'activité neuronale de la population neuronale cible.

2. Système selon la revendication 1, dans lequel le premier signal électrique (402) présente une plage de largeurs d'impulsion allant de 10 ± 10 % millisecondes à 1 ± 10 % seconde.

3. Système selon la revendication 1, dans lequel la première direction va de la partie caudale vers la partie rostrale.

4. Système selon la revendication 1, dans lequel les instructions, lorsqu'elles sont exécutées, amènent le générateur de signaux (101) à délivrer la phase initiale de telle sorte qu'un courant circule dans une seconde direction le long de la moelle épinière du patient pendant la phase initiale, la seconde direction étant associée à la suppression de l'activité neuronale de la population neuronale cible.

5. Système selon la revendication 4, dans lequel la seconde direction va de la partie rostrale vers la partie caudale.

6. Système selon la revendication 1, dans lequel la population neuronale cible comprend des neurones non adaptatifs.

7. Système selon la revendication 1, dans lequel : le premier ensemble d'électrodes (A) comprend une première électrode (321) et une deuxième électrode (324) ; le second ensemble d'électrodes (B) comprend une troisième électrode (322) et une quatrième électrode (323) ; et la troisième électrode (322) et la quatrième électrode (323) sont positionnées entre la première électrode (321) et la deuxième électrode (324) le long du dispositif de délivrance de signaux (110).

8. Système selon la revendication 1, dans lequel la population neuronale cible comprend des neurones dans la moelle épinière du patient (190), et dans lequel le dispositif implanté de délivrance de signaux (110) est conçu pour être positionné à proximité de la moelle épinière (191) du patient (190).

9. Système selon la revendication 1, dans lequel la population neuronale cible comprend un ou plusieurs nerfs périphériques du patient (190), et dans lequel le dispositif implanté de délivrance de signaux (110) est conçu pour être positionné à proximité du ou des nerfs périphériques.

10. Système selon la revendication 9, dans lequel le ou les nerfs périphériques comprennent un ou plusieurs neurones qui innervent directement le tissu musculaire.

11. Système selon la revendication 9, dans lequel le ou les nerfs périphériques comprennent le nerf vague.

12. Système selon la revendication 7, dans lequel la troisième électrode (322) est positionnée entre la première électrode (321) et la deuxième électrode (324), et la deuxième électrode (324) est positionnée entre la troisième électrode (322) et la quatrième électrode (323).
